# EUROPEAN PATENT APPLICATION

(11) **EP 1 547 526 A1**
(43) Date of publication of application: **29.06.2005**
(21) Application number: 03079163.6
(22) Date of filing: 23.12.2003
(51) Int. Cl.: A61B 17/00, A61B 17/11

(54) **Operation element, operation set and method for use thereof**

(71) Applicant: UMC Utrecht Holding B.V., 3584 CK Utrecht (NL)
(72) Inventor: de Winter, Erwin, 2610 Wilrijk (BE); Tulleken, Cornelis Antonius Franciscus, 3581 HN Utrecht (NL); Mansvelt Beck, Hendricus Jacobus, 3721 XK Bilthoven (NL); Bremmer, Jochem-Paul, 3511 VP Utrecht (NL)
(74) Representative: Prins, Adrianus Willem, Mr. Ir.

(57) **Abstract**

Operation element for use in by-pass surgery, comprising a ring shaped element having windings, preferably at least one-and-a-half winding, at least partly of wire material in close proximity, provided with at least one sharpened end.

## Description

The invention relates to an operation element. The invention especially relates to an operation element for use in by-pass surgery for connecting a shunt to a blood vessel such as a vein, artery, vessel or the like.

In by-pass surgery an opening has to be provided in a vessel to which a shunt has to be connected for forming a by-pass to for example a blood clogging, a blockage of said vessel or the like. If the flow of blood through the relevant vessel can not temporarily be stopped, the shunt has to be connected while the blood still flows through said vessel, such as non-occlusive anastomosis. Such is for example often necessary in by-pass surgery in or near the brain, which is a form of surgery on a very small scale.

In brain surgery it is commonly known to use a part of a donor vessel, for example taken from a patient's leg, as a shunt. This shunt is sewn to the outside of a vessel to which it is to be connected, by suture. The surgeon has to suture the free edge of the shunt to said vessel through the wall of said vessel. This is commonly to be performed deep within the skull, leaving the surgeon very little space to operate, whereas the view on the operating surface will be limited by body tissue, instruments and the surgeon. After the shunt has been sutured to said outside wall of said vessel, the wall part of the vessel within the free end of said shunt is removed, preferably using a laser. The shunt is then flushed for a short period, in order to remove any possible debris, after which the shunt is clamped shut and connected with the opposite end to an other part of the same vessel or to a different vessel, in a similar manner. Such technique is the ELANA (Excimer Laser Assisted Non-occlusive Anatomises) operation technique, which has been developed and optimised by prof. dr. C.A.F. Tulleken, University Medical Centre of Utrecht, The Netherlands and the Annemarie Tulleken Foundation, The Netherlands.

Due to the suturing and the small and restricted operating space this method is difficult and time consuming, whereas it may be prone to irregularities.

An object of the resent invention is to provide an operation element to be used in by-pass surgery as for example described here above, which prevents or alleviates at least some of the problems of the known method.

A further object of the invention is to provide such operation element, which is easy to use, especially in positioning thereof and in connecting a shunt.

A still further object of the present invention is to provide an operation element which can simplify the operating procedure, which can provide for a secure connection and is minimal invasive.

A further object is to provide for an operation element suitable for use with non-occlusive anastomosis, especially for but not limited to carnal or brain surgery.

These and other objects are provided for by an operation element according to the present invention, characterised by the features of claim 1.

An operation element according to the present invention is provided with a ring shaped element, at least partly made of wire and having a sharpened end. The ring shaped element has windings, at least partly in close proximity.

The sharpened end is designed such that it can be pricked through the wall of a blood vessel without excessive force. The ring shaped element can then be moved, in particular be rotated and/or translated such that at least part of said windings can be moved into said vessel, under said wall through which said sharpened end has been moved. Since said windings are at least partly in close proximity said wall will be clamped between said windings. This results in a close fit, enabling easy and accurate positioning of the ring shaped element on a wall of a vessel, using simple instruments such as a pincer or pliers. Part of the windings can be kept on the outside of the wall of said vessel, such that this provides a surface for connecting a shunt.

Since at least part of a winding of said ring shaped element will be positioned under said wall, the ring shaped element can be used as a working surface, similar to an anvil, which will be stable and allow the surgeon to work on.

An operation element, especially a ring shaped element thereof according to the invention preferably has at least 1.5 windings, more preferably between 1.5 and 2 windings. This means that approximately one winding can be brought under the wall inside a vessel whereas also approximately one winding can be kept on the outside of said wall. The ring shaped element is then simple in construction, easy to manufacture and will provide for approximately a full circle on top of and under said wall.

Preferably the ring shaped element has a first and second winding, connected to each other by a connecting part. The first and second winding are both substantially flat and lie parallel to each other, the connecting part being bent. This results in a ring shaped element that in itself is flat. During use the first winding can lie under said wall, inside said vessel, the second winding lying on said wall, outside said vessel. The connecting part will then stick through said wall. Due to the flat construction the wall part of the vessel to which the operation element is connected will be flattened, providing for a substantially flat wall part within a central opening of said ring shaped element.

A ring shaped element of an operation element according to the present invention is preferably circular.

In a particularly advantageous embodiment the sharpened end of said ring shaped element is bent relative to the direction of winding. This has the advantage that when positioning the ring shaped element by rotating thereof in the direction of winding after penetrating said wall with said sharpened end for the first time for bringing this into said vessel, the sharpened end will not be prone to be forced into or through the wall of said vessel again. Preferably said end is bent backward relative to the direction of winding, more preferably lying in a imaginary cylindrical surface defined by the windings.

The ring shaped element is preferably made of wire. The wire preferably has a substantially circular cross section with a diameter of less than 2 mm, preferably between 0.1 and 2 mm and more preferably approximately 0.25 mm and can be chosen depending on the intended use, that is type and place of surgery. In an alternative embodiment wire the cross section of similar size, but with a flattened portion, such that of part of windings abutting each other are flatted for increasing the contact surface and therefore the clamping force exerted on the wall of a vessel.

In a further alternative embodiment the ring shaped element can be connected to or part of a tube shaped element, such that a winding of said ring shaped element can be brought within a vessel, as described before, whereas said tube shaped element will be kept on the outside of said vessel, to which element a shunt can be connected.

The invention further relates to an operation set, comprising an operation element as described and a connecting element for connecting to said operation element. Such connecting element will enable easy connection of a shunt to said operation element, further facilitating easy use.

The connecting element preferably comprises hook shaped elements, which can hook around the ring shaped element, at least on the outside of said vessel. This enables suture free connection. Preferably a locking element can be forced over said hook shaped elements, for locking these in position around said ring shaped element.

In a further advantageous embodiment a ring is provided with a central opening, which can be enclosed within a folded back free end of a shunt to be connected to said operation element. This ring will provide stability to a folded edge formed by said folded back free end. The ring can preferably either fit inside a central opening of said ring shaped element or fit on top of said ring shaped element. A clamping element may be provided for clamping said shunt onto said ring shaped element.

The present invention further relates to a set of an operation set according to the present invention and a shunt. In the present invention a shunt can at least be a natural shunt such as part of a blood vessel, or can be artificial. The shunt preferably has an inner cross section substantially equal to the cross section of the ring shaped element, especially of a central opening thereof.

The present invention further relates to a shunt for use with an operation element or set according to the present invention, which shunt has a first end folded back and outward over a ring.

The present invention furthermore relates to a method for connecting a shunt to a blood vessel, characterised by the features of claim 19.

With such method a surgeon can relatively easy prepare a vessel for connecting a shunt, even in non-occlusive anastomosis. Since the ring shaped element can be position with a limited set of relatively simple tools, the surgeon has relatively much freedom to operate. He will not be hampered by for example suturing devices and the like. The ring shaped element can be placed suture free. The surgeon can freely decide how to connect a shunt to said operation element.

In a method according to the present invention preferably a ring shaped element is used having a first and second winding, connected by a connecting part, whereby the first winding is brought under a wall inside a vessel whereas the second winding is kept on the outside of said wall. The wall is clamped between the first and second winding, leaving only the connecting part traversing said wall. The ring shaped element can to that end be rotated around a longitudinal axis.

Preferably a ring shaped element is used having a sharpened end bent backward relative to the direction of winding, such that the ring shaped element is first moved in a first direction, against the winding direction, for bringing the end through said wall, after which the ring shaped element is rotated in the opposite direction, the winding direction, for bringing the first winding under said wall, within said vessel. To that end the ring shaped element is rotated for example over an angle of more than 270°, more specifically over an angle of approximately between 320 and 360°.

In a preferred embodiment a method according to the present invention is further characterised by the features of claim 23.

This method provides for a stable end of the shunt to be connected to the ring shaped element, compatible to said ring shaped element is dimensions and form. This enables relatively easy fluid tight connection of the ring shaped element and the shunt.

In a still further preferred embodiment the wall part of said vessel extending within a central opening of the ring shaped element, which wall part may be substantially flat, is sucked outward a little, into said central opening, before removal thereof. This leads to an opening in the wall within said central opening with a more desirable shape and dimensions, having smoother edges enhancing uninhibited blood flow through the shunt and blood vessel.

In the sub claims advantageous embodiments are given of the present invention. For a better understanding of the present invention embodiments thereof will be described hereafter, with reference to the drawings. These show:
Fig. 1A - D on an enlarged scale an operation element according to the present invention form various angles;
Fig. 2A - G different steps in the attachment of a shunt to a vessel according to the present invention;
Fig. 2H in cross section part of a connection made according to fig. 2A - 2G;
Fig. 3A - D in elevated view, partly broken away, top view and two perpendicular side views respectively an alternative embodiment of a operation element shaped element according to the present invention;
Fig. 4 in a cross section according to fig. 3C an operation set on a blood vessel, in further enlarged scale: and
Fig. 5 in perspective view, partly broken away, a plug and receiver of an embodiment according to fig. 3 and 4, as well as the plug combined with a shunt.

In the following description identical or corresponding components will be referred to with the same or corresponding reference signs. The embodiments shown are only shown by way of example and should by no means be understood as limiting the scope of protection in any way. Especially combinations of features of the various embodiments are considered falling under the present invention. Any reference to sizes and dimensions, shapes, materials or known apparatus used in the description should be considered as examples only, unless specifically stated otherwise.

As of 1993 the ELANA operation technique has become available, allowing non-occlusive bypass surgery, especially in the brain. In this technique an excimer laser is used for opening the wall of a blood vessel after connecting a shunt to said wall. To this end a metal ring is sutured to said wall, to which ring then an end of said shunt is connected by further suturing. The end of said laser is then introduced into said shunt, facing the wall within said ring, which wall part is then removed by said laser. This operation technique is very successful but has the main disadvantage that connecting said ring to said wall and connecting said shunt to said ring, both by suturing is cumbersome, especially with small sizes and in restricted operating areas such as in brain surgery. The ELANA technique as discussed in Tulleken CA, Verdaasdonk RM, Beck RJ, et al: The modified excimer laser-assisted high-flow bypass operation. Surg.Neurol. 46:424-429, 1996; and Tulleken CA, Verdaasdonk RM, Mansvelt Beck HJ: Nonocclusive excimer laser-assisted end-to-side anastomosis. Ann.Thorac.Surg. 63:S138-S142, 1997 is considered to be enclosed herein by reference.

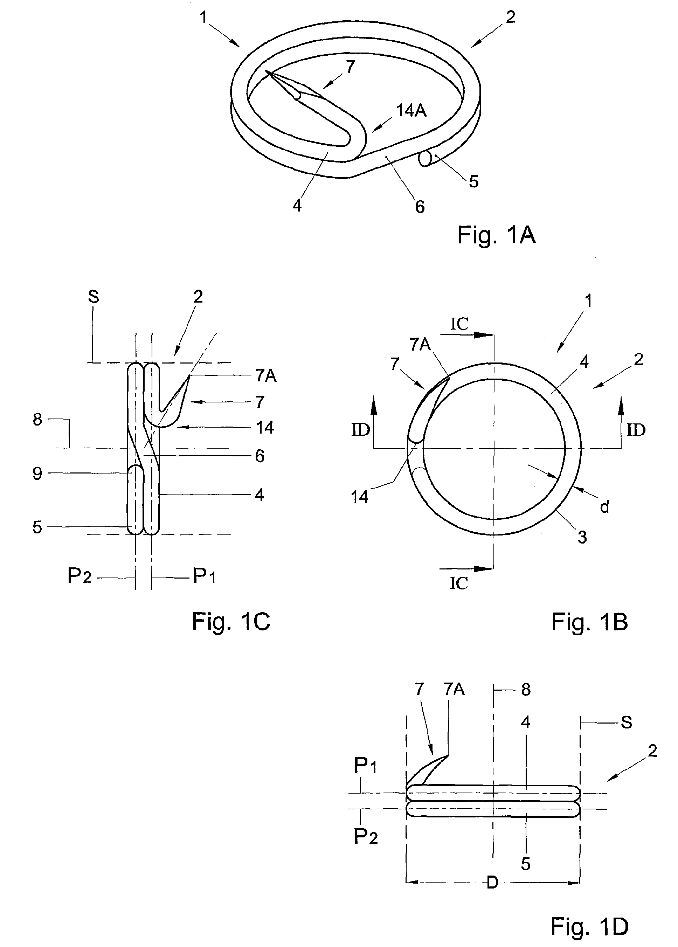

Fig. 1A shows in perspective view an operation element 1 according to the present invention, whereas fig. 1B shows the operation element from above. Fig. 1C and 1D show the operation element in perpendicular side views according to the arrows IC - IC and ID - ID as shown in fig 1B. This operation element 1 comprises or is constituted by a ring shaped element 2. This ring shaped element is made of metal wire 3 having for example a circular cross section with a diameter d of several micrometer, whereas the ring shaped element 1 as such has a diameter D of a few millimetre. The sizes d and D are dependent on the operation to be performed and especially the blood vessel onto which said operation element 1 is to be used. By way of example only, the diameter d of the wire can for example be between 0 and 2 mm, especially between 0.1 and 2 mm and the diameter D of the ring shaped element 1 can for example be between 1 and 40 mm, especially 1 and 25 mm. For brain surgery for example a ring having a diameter D of approximately 2.8 mm and a diameter d of the wire of approximately 0.25 mm can be used. The wire is made of a bio compatible material, FDA approved, and can for example be made of platinum or other metal, plastic or the like.

The ring shaped element 2 comprises more than one winding, preferably at least one-and-a-half winding. In the embodiments shown the ring shaped element 2 comprises approximately two windings. More specifically the ring shaped element 2 comprises a first part 4 and a second part 5, both substantially flat, lying approximately parallel to each other and comprising a little less than a full circle. Each part 4, 5 encloses for example an angle of 320° to 340°. The first part 4 is connected to the second part 5 by a connecting part 6 formed by a substantially elongated S-shaped wire element.

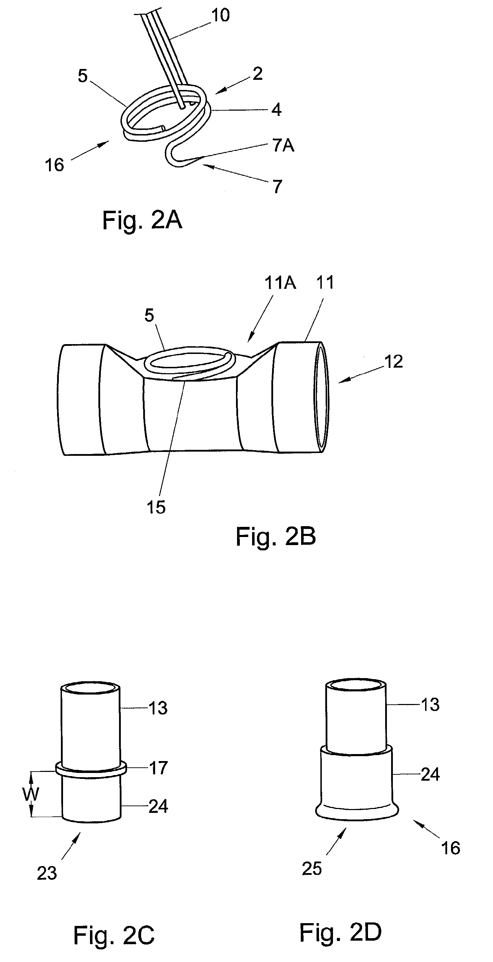

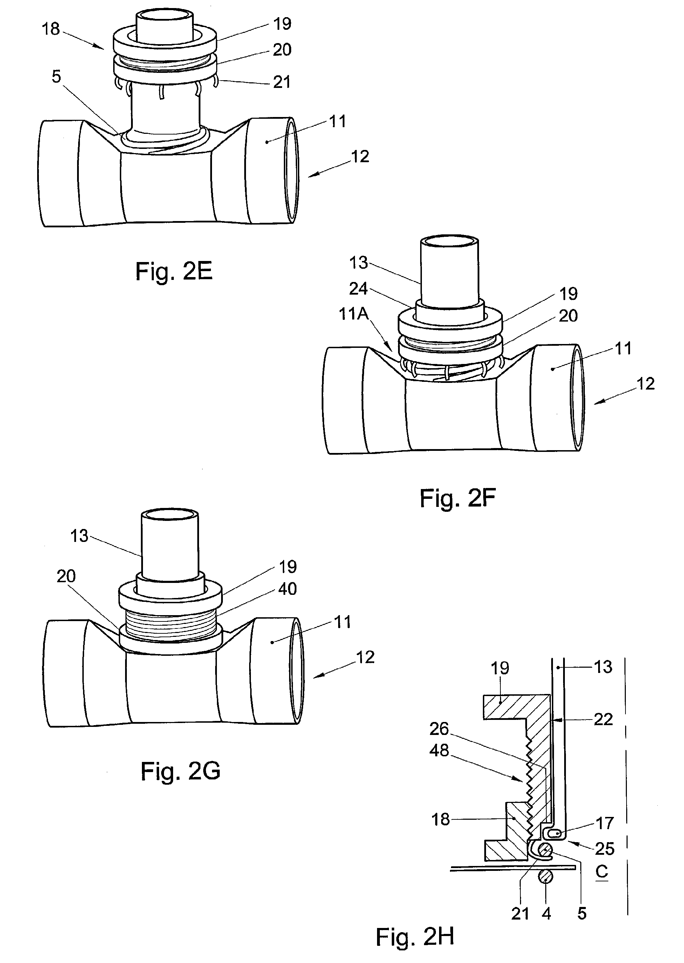

The first part 4 has a free end 7, which has a sharpened point 7A, such that it can relatively easy penetrate the wall 11 of a blood vessel 12 (fig. 2) The free end 7 is bent back and away from the second part 5. The ring shaped element 2 has a central axis 8 extending perpendicular to the planes P1 and P2 defined by the first 4 and second part 5 respectively, such that the said parts 4, 5 define a substantially cylindrical surface S on the outside of said ring shaped element 2. The said free end 7 is preferably bent such that it lies parallel to or in said outer surface S but it may also cross said surface S. The free end 7 encloses an angle α with said planes P1, P2, preferably an acute angle, more specifically an angle of between 10 and 60°. The free end 9 of the second part 5 is preferably blunt.

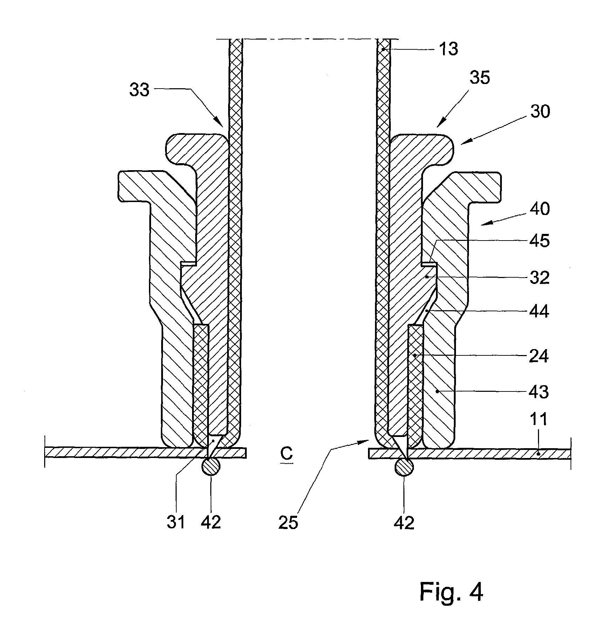

The material used for the ring shaped element 2 is preferably resilient, such that said first and second part 4, 5 are clamped onto each other. Due to the shape of the cross section of the wire the contact surface between said two parts 4, 5 is preferably very small, more preferably a line-contact, such that a relatively high clamping force is obtained. The purpose thereof will be explained hereafter, especially with reference to fig. 2 and 4.

In operation the operation element will be connected to a blood vessel as follows. The ring shaped element 2 will be picked up by an operation tool, for example a pincer 10, for example substantially parallel to the axis 8, distanced from the sharp free end 7. Said sharp free end 7 is forced through the wall 11 of a blood vessel 12 at a position where a shunt 13 such as a donor blood vessel or artificial vessel has to be connected. The ring shaped element is then forced into the direction of said wall 11 further, until the bent 14 between the free end 7 and the first part 4 reaches said wall 11. During this first part the ring shaped element 2 is rotated slightly around the axis 8 and/or translated in a first direction, in the embodiment shown counter-clockwise.

When the said bent 14 is introduced in said wall 11 the ring shaped element 2 will be rotated around said axis 8 in the opposite second direction, in the embodiment shown clockwise, such that said wall 11 is forced between the first and second part 4, 5. The ring shaped element 2 will preferably be rotated over such an angle that the connecting part 6 will be next to the opening 15 in the wall 11 through which the free end 7 is introduced or may be at least partly be introduced into said opening 15. This rotation leads to the effect that the first part 4 will be introduced into said blood vessel 12, lying against the "inside" of said wall 11, whereas the second part 5 will be maintained outside said vessel 12, lying against the "outside" of said wall 11. The wall 11 itself will thus be clamped between said first 4 and second part 5. Due to the free end 7 being bent backward the blunt side 14A of the bent 14 will be directed forward seen in the direction of rotation (clockwise) for introducing the first part 4 into said vessel 12. This prevents the free end 7 from puncturing other parts of the wall 11.

Due to this construction and method, the ring shaped element 2 will be firmly held in position and form a firm basis for attachment of the shunt 13. No suturing is necessary for positioning said ring shaped element 1 and placing the ring shaped element 1 is a relatively simple procedure.

A shunt 13 can be connected to said ring shaped element 2, especially to the second part 2 thereof in a known manner, using suturing and the basics of the ELANA technique or any other suitable technique. However, preferably the ring shaped element forms part of a set 16, further comprising a second ring 17 and a connecting element 18. The second ring 17 preferably has a diameter approximately equal to the diameter D of the ring shaped element 2 and/or to the outer diameter of the shunt 13 to be connected. The connecting element 18 comprises an upper ring 19 and a lower ring 20. The lower end of the upper ring 19 is provided with a number of hook shaped elements 21 of a resilient or pivotable nature. The hook shaped elements 21 are placed and dimensioned such that in a first position (as shown in fig. 2E) that they can be slide over the outer periphery of the second part 5 of the ring shaped element 2, as will be discussed hereafter. The lower ring 20 can be forced down along the upper ring 19, over the hook shaped elements 21, forcing the hook shaped element 21 inwards towards a second position (shown in fig. 2F and G) The lower ring 19 can then be fixed in said position, for holding the hook shaped elements in said second position. The upper ring 19 has an inward facing flange 26 at its lower end, which flange 26 is ring shaped and has a central opening 22 corresponding approximately to the inner diameter of the second ring 17, such that said second ring 17 can not pass said flange and thus can not pass said connecting element through the upper and lower rings 19, 20.

A set 16 can be used as follows.

The ring shaped element 2 (fig. 2A) is positioned on the wall 11 of a vessel 12 as described here above (fig. 2B) Due to placement of the ring shaped element 2 the relevant wall part 11A of the vessel 12 is flattened. The second ring 17 is placed over a free end 23 of a shunt 13, at a distance W from said free end (fig. 2C) forming a skirt 24 The skirt 24 is then folded back over said second ring 17 (fig. 2D) enclosing the second ring 17. Thus a folded end 25 is formed, supported by the second ring 17. Said folded end 25 is position against the second part 5 of the ring shaped element 2. The connecting element 18 is moved over the shunt 13 towards the ring shaped element 2, the lower ring 20 facing said ring shaped element 2 (fig. 2E) The connecting element 18 is forced down against the ring shaped element, such that the hook shaped elements 21 are next to the second part 5 of the ring shaped element 2, the upper ring 19 resting on the lower ring 20 (fig. 2F) Finally the lower ring 20 is forced down along the upper ring 19, for example by intermeshing screw threads 48 or a push fit or the like, such that the hook shaped element 21 are forced at least partly under the second part 5, locking the connecting element 18 in place. At the same time the flange 26 is forced against the second ring 17 with the wall of the shunt 13 in between, pressing the folded end 25 of the shunt in a fluid, especially blood tight connection. In this position the upper and lower rings 19, 20 are locked and the connection is made, as shown in fig. 2H.

After making said connection the ELANA technique is used for removing the wall part within said ring shaped element 2, thereby forming a connecting opening C and flushing, after which the shunt is temporarily closed of by a clamp (not shown).

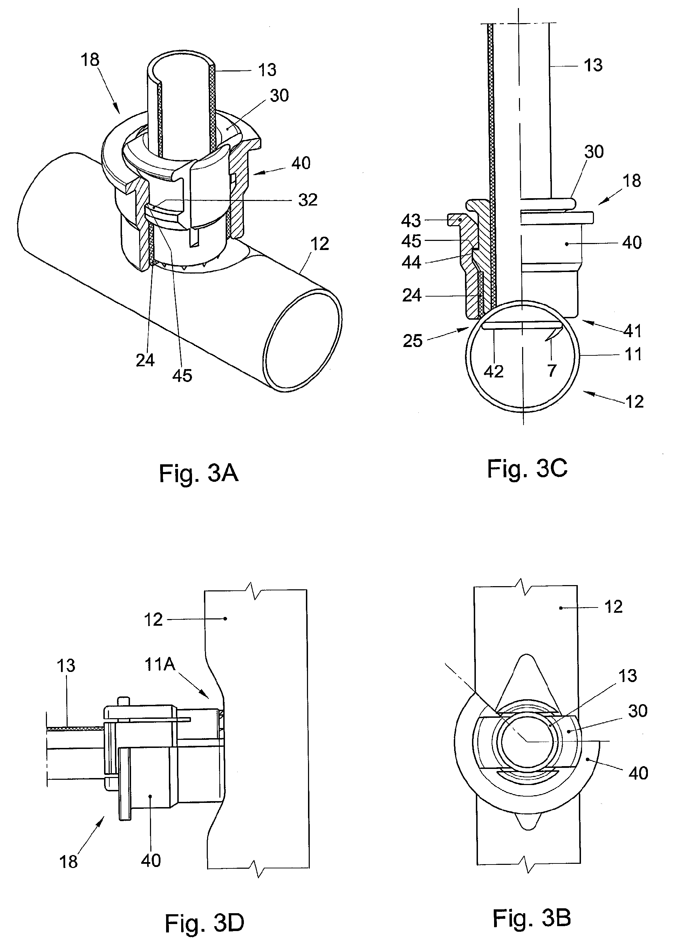

Fig. 3 - 5 disclose a further embodiment of an operation set according to the present invention, which will be described along with an alternative embodiment of a method for applying such.

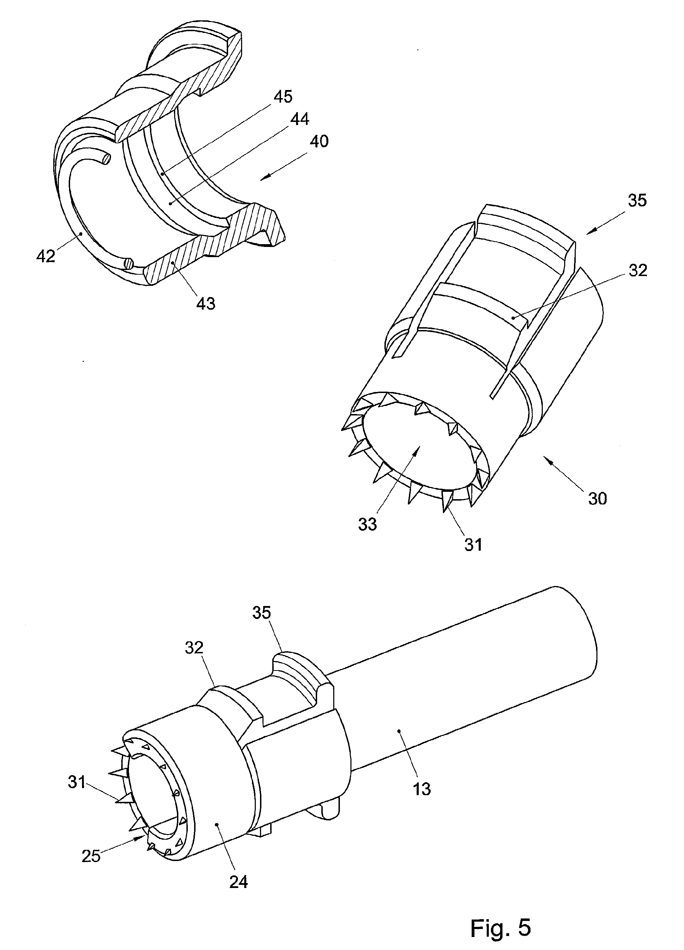

In fig. 3A a perspective view of a second embodiment of a set 16 according to the present invention, partly cut away, positioned on a vessel 12 such as a vein or artery. In fig. 3B a top view thereof is shown, in fig. 3C and 3D to side views. In fig. 4 a cross section is given. Fig. 5 shows the shunt 13 of a set according to fig. 3 and 4, with the connecting element 18. It should be emphasized that "connecting element" and "ring shaped element" are referred to a single elements, these may obviously comprise several parts or elements.

In the embodiment of fig. 3 - 5 again a ring shaped element 2 is provided on a wall 11 of a vessel 12, as shown in fig. 4. In this embodiment the ring shaped element 2 is provided in the form of a substantially cylindrical receiver 40 provided at a first, lower end 41 with a ring 42 comparable to the first part 4 of a ring shaped element according to fig. 1, including a sharpened end 7. The cylindrical wall 43 of the receiver 40 functions as the second part 5 according to fig. 1. On the inside of the cylindrical wall 43 a groove 44 is provided having a flat upper ridge 45. The cylindrical receiver 43 can be positioned on and connected to a wall 11 of a vessel 12 in a manner similar to the method as described with reference to fig. 1 and 2, by forcing the ring 42 through and under the wall 11 of the vessel 12, thereby partly flattening said wall 11.

In this embodiment in stead of the second ring 17 an inner connecting element 30 or plug is provided, having spike like elements 31 at a lower end and an outwardly extending click-ridge 32 spaced apart from said spike like elements 31. Said ridge 32 may be provided at least partly on two resilient fingers 35. The shunt 13 is introduced through a channel shaped opening 33 through said inner connecting element 31 and the free end 23 of said shunt 13 is folded back onto the outside of said inner connecting element 31 to just below said ridge 32, forming said skirt 24. The spike like elements 31 will penetrate through the folded end 25 of said shunt 13. This position is shown in fig. 5, below, whereas the separate parts 30 and 40 are shown there above.

When the shunt 13 is prepared as described, the inner connecting part 31 with the shunt 13 is forced into the receiver40, connected to the vessel 12 as described, having the spike like elements 31 facing forward into the direction of the wall 11. The spike like elements 31 are forced into and possibly through the wall 11 within the ring shaped element 40, preventing rotation and partly loosening said wall part 11. The click ridge 32 will be forced into said groove 44 under the ridge 45, thereby pressing the folded end 25 against the wall 11 and indirectly against the ring 42, forming a liquid tight connection and at the same time fixing the plug 30 in the receiver 40. Said ELANA technique or other suitable techniques may then be used for forming the connecting opening C.

It will be clear that a locking mechanism as disclosed in fig. 3 - 5 may be used with a ring shaped element 2 according to fig. 1, whereby the receiver may be provided with the hook shaped elements or similar connecting parts for connecting to the ring shaped element 2. Or vice versa, whereas connecting elements and techniques as described could also be used with the known ELANA technique using sutured rings.

The present invention is by no means limited to the embodiments of the present invention as have been shown by way of example. Many variants are possible within the scope of the present invention as defined by the claims.

A ring shaped element mat be of a different design and can have more then the number of windings shown. It could for example be part of a wound spring. In an embodiment similar to fig. 3 - 5 the ring shaped element could also be connected to the plug, such that the folded back end of the shunt and the wall of the vessel will be enclosed between the clamping parts of the ring shaped element, such as the lower wall end of the plug and the ring 41. The receiver would then lock the shunt against the plug 30. Other locking means for the connecting elements may be used, for example bayonet means, gluing means and the like. In theory the ring shaped element can have any form, dimension, size and shape. A substantially circular shape is favourable since it can then easily be rotated. An operation element, set or method according to the present invention can also be used in other areas of operation the by-pass surgery in the brain.

## Claims

1. Operation element for use in by-pass surgery, comprising a ring shaped element having windings, preferably at least one-and-a-half winding, at least partly of wire material in close proximity, provided with at least one sharpened end.

2. Operation element according to claim 1, comprising more than one-and-a-half winding but less than two windings.

3. Operation element according to claim 1 or 2, wherein said ring shaped element comprises a central axis and defines a substantially cylindrical outer surface, wherein said sharpened end is bent such that it lies substantially in or parallel to said outer surface.

4. Operation element according to claim 3, wherein said sharpened end is bent back into the direction of winding.

5. Operation element according to any one of claims 1 - 4, wherein the ring shaped element comprises a first part and a second part, the first and second part being substantially flat and being connected to each other by a connecting part, such that the first and second part lie substantially parallel to each other.

6. Operation element according to any one of claims 1 - 5, wherein the wire has a substantially circular cross section or a cross section with a flattened portion, such that of the ring shaped elements touching parts are said flattened portions.

7. Operation element according to any one of claims 1- 6, wherein the wire has a diameter of less than 2 mm, preferably less than 1 mm and more preferably approximately 0.25 mm.

8. Operation element according to any one of the previous claims, wherein said ring shaped element is connected to or part of a tube shaped element.

9. Operation set for connecting a shunt to a vein or artery, comprising an operation element according to any one of the previous claims and a connecting element for connection to said operation element.

10. Operation set according to claim 9, wherein said connecting element comprises at least two hook shaped elements, designed for hooking around said ring shaped element, preferably around said at least one winding.

11. Operation set according to any one of claims 9 or 10, wherein said connecting element comprises a tube having a central canal, having a cross section approximately equal to the cross section of said ring shaped element.

12. Operation set according to any one of claims 9 - 11, wherein the set further comprises a second ring having a central opening, preferably having a cross section substantially equal to the cross section of said ring shaped element.

13. Operation set according to any one of claims 9 - 12, wherein furthermore a clamping element is provided.

14. Operation set according to any one of claims 9 - 13, wherein furthermore an instrument is provided for engaging said ring shaped element and turning said ring shaped element over at least 270°

15. Set of an operation set according to any one of claims 9 - 14 and a shunt.

16. Set according to claim 15, wherein said shunt has an internal cross section substantially equal to the cross section of a ring shaped element of the operation set.

17. Set according to claim 15 or 16, wherein said shunt has a first end which is folded back over a ring.

18. Shunt, comprising an end folded back over a ring, such that said ring is enclosed between an outside of said shunt and said end folded back.

19. Method for connecting a shunt to a blood vessel such as an artery or vein, comprising the steps of:
- sticking a front end of a ring shaped element through the wall of a vessel, said ring shaped element having more than one winding;
- moving said ring shaped element such that at least part of at least one winding is brought under said wall, whereas an other part of the ring shaped element is kept on the opposite outer side of said wall;
- whereby said part of said wall is clamped between said part of said winding under said wall ad said part of said ring shaped element on the outer side of said wall.

20. Method according to claim 19, wherein a ring shape element is used having a first winding and a second winding, connected by a connecting part, whereby said first winding is positioned under said wall and said second winding is positioned on said outside of said wall, the connecting part extending through said wall.

21. Method according to claim 19 or 20, wherein the ring shaped element is rotated around a longitudinal axis for bringing said part of said at least one winding under said wall.

22. Method according to any one of claims 19 - 21, wherein a shunt is connected to said part of the ring shaped element on the outside of said wall.

23. Method according to claim 22, wherein a shunt is used having a substantially tube like form, wherein a first end of said shunt is folded back over a ring, such that said ring is enclosed by said shunt, forming a folded edge supported by said ring, whereby said folded edge is positioned against said ring shaped element on the outside of said wall and is subsequently connected to said ring shaped element in a fluid tight position.

24. Method according to claim 22 or 23, wherein a clamping element is moved over the outside of said shunt for clamping said folded edge to said ring shaped element.

25. Method according to any one of claims 19 - 24, wherein a shunt is connected to said ring shaped element, after which part of said wall extending within a central opening of said ring shaped element is removed, preferably by laser.

26. Method according to claim 25, wherein said wall part extending within said central opening is sucked into said central opening forming a convex surface, before removing of said part of said wall.
